# EUROPÄISCHE PATENTSCHRIFT

(11) **EP 3 261 570 B1**
(45) Veröffentlichungstag und Bekanntmachung des Hinweises auf die Patenterteilung: **04.03.2020**
(21) Anmeldenummer: 16702373.8
(22) Anmeldetag: 26.01.2016
(51) Int. Cl.: A61B 34/20, A61B 17/70, A61B 90/00, A61B 34/10

(54) **MEDIZINISCHES INSTRUMENTARIUM**
MEDICAL INSTRUMENT
INSTRUMENT MÉDICAL

(30) Priorität: 26.02.2015 DE 102015102768
(43) Veröffentlichungstag der Anmeldung: 03.01.2018
(73) Patentinhaber: Aesculap AG, 78532 Tuttlingen (DE)
(72) Erfinder: KOZAK, Josef, 78532 Tuttlingen (DE); BEGER, Jens, 78532 Tuttlingen (DE)
(74) Vertreter: Hoeger, Stellrecht & Partner Patentanwälte mbB
(86) Internationale Anmeldenummer: PCT/EP2016/051582
(87) Internationale Veröffentlichungsnummer: WO 2016/134904

(56) Entgegenhaltungen:
- EP-A1- 2 910 206
- WO-A1-2011/020505
- WO-A2-03/020146
- WO-A2-2013/164770
- DE-A1-102008 022 254
- DE-U1-202015 100 313

## Beschreibung

Die Erfindung betrifft ein medizinisches Instrumentarium, umfassend ein medizintechnisches Navigationssystem.

Ein derartiges Instrumentarium mit Navigationssystem kommt bei navigationsgestützten chirurgischen Eingriffen zum Einsatz, um den Operateur zu unterstützen. Über das Navigationssystem können mittels medizinischer Markiereinrichtungen markierte charakteristische Punkte oder Landmarken an einem Patienten aufgenommen werden. Bekannt ist es auch, chirurgische Instrumente oder Implantate mit Markiereinrichtungen zu versehen, deren Lage und/oder Orientierung relativ zum Patienten und speziell zu einer daran angeordneten Referenzmarkierung bestimmt werden.

Medizintechnische Navigationssysteme bewähren sich in der Praxis. Sie erfordern jedoch einen nicht unerheblichen Platzbedarf und verursachen nicht vernachlässigbare Kosten.

Ausführungsformen chirurgischer Instrumentarien mit medizintechnischen Navigationssystemen sind in der WO 2013/164770 A2, der WO 2011/020505 A1 und in der nachveröffentlichten (Art. 54(3) EPÜ) EP 2 910 206 A1 beschrieben.

Die WO 2013/164770 A2 beschreibt ein Instrumentarium, das ähnlich zur beanspruchten Erfindung ist, jedoch keine zwei oder mehr medizinische Markiereinrichtungen aufweist sowie keine Raumvektoren ermittelt.

Die EP 2 910 206 A1 beschreibt ebenfalls ein ähnliches Instrumentarium, das jedoch keine Beleuchtungseinheit sowie retroreflektierende Markierelemente offenbart.

Die Druckschriften WO 03/020146 A2 und DE 10 2008 022 254 A1 offenbaren Ausführungsformen von Markierelementen für die optische Koordinatenerfassung.

Die DE 20 2015 100 313 U1 beschreibt die optische Erfassung von in codierter Form vorliegenden Informationen mittels eines medizintechnischen Navigationssystems.

Aufgabe der vorliegenden Erfindung ist es, ein medizinisches Instrumentarium umfassend ein medizintechnisches Navigationssystem bereitzustellen, das konstruktiv einfach ausgestaltet und nach Möglichkeit kostengünstig herstellbar ist.

Diese Aufgabe wird erfindungsgemäß durch ein medizinisches Instrumentarium mit den Merkmalen von Anspruch 1 gelöst.

Beim erfindungsgemäßen Instrumentarium kommt ein auf benutzerfreundliche Weise einsetzbares und handhaltbares integriertes medizintechnisches Navigationssystem zum Einsatz. Unter "integriert" kann vorliegend insbesondere verstanden werden, dass die Komponenten des Navigationssystems - Datenverarbeitungseinheit, Anzeigeeinheit, Beleuchtungseinheit - in einem gemeinsamen Gehäuse des Navigationssystems aufgenommen sind, und dass das Navigationssystem bevorzugt nur ein Gehäuse umfasst. Beispielsweise kann der Operateur das Navigationssystem in jeder beliebigen Orientierung zu einer zu erfassenden Markiereinrichtung positionieren, so dass sich das Navigationssystem nicht nur als handhabungsfreundlicher erweist als herkömmliche, raumfeste medizintechnische Navigationssysteme, sondern auch als vielseitiger. Mittels der Beleuchtungseinheit kann Licht in Richtung der zu erfassenden Markiereinrichtung ausgesandt werden, das von deren Markierelementen reflektiert und von der Kamera der Erfassungseinheit empfangen werden kann. Durch die Beleuchtungseinheit kann sichergestellt werden, dass die Markiereinrichtung in nahezu jeder beliebigen Relativorientierung des Navigationssystems und der Markiereinrichtung zuverlässig erfasst werden kann. Durch die Integration der Komponenten in einem handhaltbaren Gerät können ferner Herstellungskosten für das Instrumentarium gering gehalten werden.

Die Kamera der Erfassungseinheit erstellt insbesondere ein Bild oder Bildfolgen von der Umgebung und einer darin angeordneten Markiereinrichtung. Bildverarbeitungsalgorithmen ermöglichen es der Datenverarbeitungseinheit, das Bild oder die Bilder der Erfassungseinheit zu analysieren und die Lage und/oder Orientierung der Markiereinrichtung zu erkennen. Über die Markiereinrichtung wird insbesondere, darauf wird nachfolgend noch eingegangen, ein Referenzkoordinatensystem definiert. Im Referenzkoordinatensystem können weitere charakteristische Punkte, Bezugspunkte oder Landmarken unter Markierung durch zusätzliche Markiereinrichtungen erfasst werden. Hierbei ist es nicht erforderlich, das Navigationssystem relativ zu den Markiereinrichtungen räumlich zu fixieren.

Das Instrumentarium weist zwei oder mehr medizinische Markiereinrichtungen auf, die jeweils eine Mehrzahl von Markierelementen umfassen, die das Licht der Beleuchtungseinheit retroreflektierend ausgestaltet sind. Beispielsweise sind die Markierelemente insbesondere zur Retroreflexion von sichtbarem Licht optimiert, zum Beispiel im Bereich von 400 nm bis 800 nm.

Günstig ist es, dass das Instrumentarium zwei oder mehr Markiereinrichtungen aufweist und dass mit der Datenverarbeitungseinheit ein Raumvektor zwischen von den Markiereinrichtungen referenzierten Bezugspunkten ermittelbar ist, wobei eine der Markiereinrichtungen als Referenzmarkiereinrichtung zur Definition eines Referenzkoordinatensystems heranziehbar ist. Im Referenzkoordinatensystem, das über eine Markiereinrichtung definiert wird, können die Lage und die Orientierung der weiteren Markiereinrichtung bestimmt werden. Von den Markiereinrichtungen referenzierte Bezugspunkte können dadurch über einen Raumvektor verbunden werden. Dies ist unabhängig von der Position des Navigationssystems relativ zu den Markiereinrichtungen möglich. Dadurch ist dem Operateur die Möglichkeit gegeben, das Navigationssystem frei zu positionieren, damit er möglichst gute Sicht auf das Operationsfeld und die Markiereinrichtungen hat.

Vorteilhafterweise sind die Markiereinrichtungen simultan mit der Erfassungseinheit erfassbar, und der Raumvektor ist mit der Datenverarbeitungseinheit anhand eines und insbesondere nur eines Bildes der Kamera ermittelbar. Günstig ist es, dass das Navigationssystem eine Sensoreinheit aufweist zum Bereitstellen eines Signals über die Neigung des Navigationssystems in mindestens einer Raumrichtung eines absoluten Bezugssystems, und dass mit der Datenverarbeitungseinheit die Orientierung des Raumvektors im absoluten Bezugssystem ermittelbar ist. Dadurch können Lage- und Orientierungsdaten aus dem Referenzkoordinatensystem der Markiereinrichtung in das absolute Bezugssystem und umgekehrt transformiert werden. Dies erlaubt es zum Beispiel, Achsen und Ebenen mit anatomischen Landmarken ohne a priori-Kenntnis der Lage des Patienten im absoluten Bezugssystem zu ermitteln.

Anhand der Orientierungen einer Mehrzahl von zwei oder mehr Raumvektoren kann eine Neigung einer durch diese definierten Ebene, etwa der Beckeneingangsebene, relativ zu einer Bezugsebene, insbesondere einer Horizontalebene, im absoluten Bezugssystem ermittelt werden.

Besonders vorteilhaft ist es, wenn das Navigationssystem als Smartphone oder als Tablet-Computer ausgestaltet ist. In der Datenverarbeitungseinheit des Smartphones oder Tablet-Computers kann ein Datenverarbeitungsprogramm ausführbar sein, bei dem insbesondere unter Zuhilfenahme von Bildverarbeitungsalgorithmen die von der Kamera erstellten Aufnahmen analysiert und die Markierelemente mindestens einer Markiereinrichtung erkannt und getrackt werden.

Vorteilhafterweise umfasst das Navigationssystem die Beleuchtungseinheit, um eine konstruktiv einfache Ausgestaltung des Instrumentariums zu erzielen.

Günstig ist es, wenn die Beleuchtungseinheit mindestens eine LED-Lichtquelle aufweist.

Von der Beleuchtungseinheit ist vorzugsweise sichtbares Licht emittierbar, beispielsweise in einem Spektralbereich von ungefähr 400 nm bis ungefähr 800 nm oder einem Teil davon.

Vorteilhafterweise umfasst die Erfassungseinheit genau eine Kamera, um die konstruktive Ausgestaltung des Navigationssystems zu vereinfachen. Das Vorsehen einer Stereokamera ist nicht erforderlich. Die Datenverarbeitungseinheit kann anhand der Aufnahmen der einen Kamera mindestens eine Markiereinrichtung zuverlässig im Raum verfolgen.

Günstigerweise ist an der Anzeigeeinheit ein mit der Kamera erfassbares Bild darstellbar. Über die Verfolgung mindestens einer Markiereinrichtung hinaus können auf diese Weise zusätzliche Informationen erfasst werden. Beispielsweise kann der Operationsablauf dokumentiert werden.

Insbesondere im Zusammenhang mit der zuletzt erwähnten vorteilhaften Ausführungsform ist es günstig, wenn das Navigationssystem eine Kommunikationsschnittstelle aufweist zum Übertragen von Daten an eine externe Empfangseinheit, insbesondere Bildern, die mit der Kamera aufgenommen werden oder wurden. Lage- und/oder Orientierungsdaten mindestens einer Markiereinrichtung, die Daten charakteristischer Punkte, Bezugspunkte oder anatomischer Landmarken können ebenso übertragen werden wie Bilder der Kamera, zum Beispiel für Schulungs- oder Dokumentationszwecke.

Besonders vorteilhaft ist es, wenn das Navigationssystem im Raum frei beweglich und frei von einer mechanischen Kopplung mit einem chirurgischen Instrument oder einem Implantat ist. Das Navigationssystem ist dadurch vielseitiger einsetzbar, und die Handhabung des Instrumentariums ist der Bedienperson vereinfacht.

Günstig ist es, wenn mindestens eine Markiereinrichtung vier oder mehr Markierelemente umfasst, um die Genauigkeit der Bestimmung der Lage und/oder Orientierung der Markiereinrichtung zu erhöhen.

Als vorteilhaft erweist es sich, wenn mindestens eine Markiereinrichtung ein Halteelement aufweist, an dem die Markierelemente gehalten sind, und Kontrasterhöhungselemente, wobei den Markierelementen ein jeweiliges Kontrasterhöhungselement zugeordnet ist zum Steigern des Kontrastes zwischen den Markierelementen und dem Halteelement.

Beispielsweise sind die Kontrasterhöhungselemente als das jeweilige Markierelement umgebender Ring ausgestaltet, der eine Reflektivität für das von der Beleuchtungseinheit emittierte Licht aufweist, die geringer ist als die Reflektivität der Markierelemente. Beim Beleuchten reflektieren die Markierelemente das von der Beleuchtungseinheit ausgesandte Licht in größerem Ausmaß als die Kontrasterhöhungselemente. In den Aufnahmen der Kamera können die Bildanteile der Markierelemente dadurch von der Datenverarbeitungseinheit besser ermittelt und die Lage und/oder Orientierung der Markiereinrichtung genauer bestimmt werden.

Vorteilhafterweise weist mindestens eine Markiereinrichtung haubenförmige Schutzelemente auf, die einem jeweiligen Markierelement zugeordnet sind und die für das von der Beleuchtungseinheit emittierte Licht transparent sind. Die Schutzelemente haben zum Beispiel eine kugelkalottenförmige Gestalt und sind optisch transparent für sichtbares Licht. Die Markierelemente werden durch die Schutzelemente vor Verschmutzung geschützt, zum Beispiel durch Blut.

Bei einer vorteilhaften Ausführungsform umfasst das Instrumentarium zwei oder mehr zu referenzierende Referenzelemente, und die Markiereinrichtungen sind vorzugsweise selektiv mit den Referenzelementen koppelbar, und anhand der Datenverarbeitungseinheit ist die Relativposition der Referenzelemente im Referenzkoordinatensystem ermittelbar. Eine jeweilige Markiereinrichtung kann in räumlich definierte Position zu einem Referenzelement gebracht werden. Dadurch kann die Lage und Orientierung des Referenzelementes im Referenzkoordinatensystem ermittelt werden. Indem eine Markiereinrichtung an einem der Referenzelemente verbleibt und mit der weiteren Markiereinrichtung weitere zu referenzierende Referenzelemente markiert werden, kann die Relativposition der Referenzelemente, etwa in Gestalt eines offenen oder geschlossenen Polygonzugs, ermittelt werden.

Es kann vorgesehen sein, dass die Markierelemente mittelbar oder unmittelbar mit den Referenzelementen koppelbar sind.

Die Referenzelemente sind zum Beispiel Knochenschrauben oder umfassen solche, und die Markiereinrichtungen können bevorzugt mit Verlängerungselementen, insbesondere Verlängerungstuben, vorzugsweise perkutan mit den Knochenschrauben koppelbar sein. Dies erlaubt es, die Relativpositionen der Knochenschrauben bevorzugt perkutan zu bestimmen.

Von Vorteil ist es, wenn das Instrumentarium ein Verbindungselement aufweist zum Verbinden der Referenzelemente, und wenn von der Datenverarbeitungseinheit die Geometrie des Verbindungselementes ermittelbar ist, um die Referenzelemente zu verbinden, vorzugsweise wenn an der Anzeigeeinheit ein Hinweis betreffend das Verbindungselement bereitstellbar ist. Sind die Relativpositionen der Referenzelemente bekannt, kann die Datenverarbeitungseinheit errechnen, was für ein Verbindungselement erforderlich ist, um die Referenzelemente miteinander zu verbinden. Ein diesbezüglicher Hinweis zur Auswahl oder zum Formen eines geeigneten Verbindungselementes kann an der Anzeigeeinheit dargestellt werden.

Günstigerweise ist das Verbindungselement mit einer der Markiereinrichtungen koppelbar, und von der Datenverarbeitungseinheit sind bevorzugt die Lage des Verbindungselementes relativ zu den Referenzelementen ermittelbar und einem Benutzer Hinweise an der Anzeigeeinheit zum Führen des Verbindungselementes bereitstellbar. Über das Navigationssystem und dessen Anzeigeeinheit kann der Benutzer angeleitet werden, die Referenzelemente mit dem Verbindungselement zu verbinden. Hierzu ist das Verbindungselement mit einer Markiereinrichtung koppelbar oder gekoppelt, beispielsweise ist die Markiereinrichtung an einem Implantationswerkzeug des Verbindungselementes festgelegt, ebenso wie das Verbindungselement. Durch Verfolgen des Verbindungselementes im Referenzkoordinatensystem wird dem Operateur das Verbinden der Referenzelemente miteinander erheblich vereinfacht. Dies erlaubt insbesondere eine einfache perkutane Verbindung der Referenzelemente miteinander.

Das Verbindungselement kann beispielsweise ein Stab sein, und eine der Markiereinrichtungen ist, wie erwähnt, an einem Einsetz- oder Implantationswerkzeug zum Führen des Stabes festgelegt oder festlegbar.

Die nachfolgende Beschreibung einer bevorzugten Ausführungsform der Erfindung dient im Zusammenhang mit der Zeichnung der näheren Erläuterung der Erfindung. Es zeigen:
- Figur 1:: eine vorteilhafte Ausführungsform eines erfindungsgemäßen In-strumentariums in schematischer Darstellung, umfassend ein handhaltbares integriertes Navigationssystem und ein chirurgisches Fixationssystem;
- Figur 2:: ein schematisches Blockdiagramm des Navigationssystems aus Fi-gur 1;
- Figur 3:: schematisch eine Teildarstellung eines Markierelementes einer me-dizinischen Markiereinrichtung;
- Figur 4:: das Instrumentarium aus Figur 1 in einer weiteren Darstellung;
- Figur 5:: eine weitere Darstellung des Instrumentariums aus Figur 1;
- Figur 6:: eine schematische Darstellung des Navigationssystems aus Figur 1 mit einem von diesem angezeigten Hinweis zur Auswahl eines Verbindungselementes;
- Figur 7:: eine weitere Darstellung entsprechend Figur 6;
- Figur 8:: das Instrumentarium aus Figur 1 beim Einsetzen des Verbindungselementes;
- Figur 9:: eine schematische Darstellung des Navigationssystems, das einen Hinweis zum Einsetzen des Verbindungselementes bereitstellt und
- Figur 10:: eine weitere Darstellung entsprechend Figur 9.

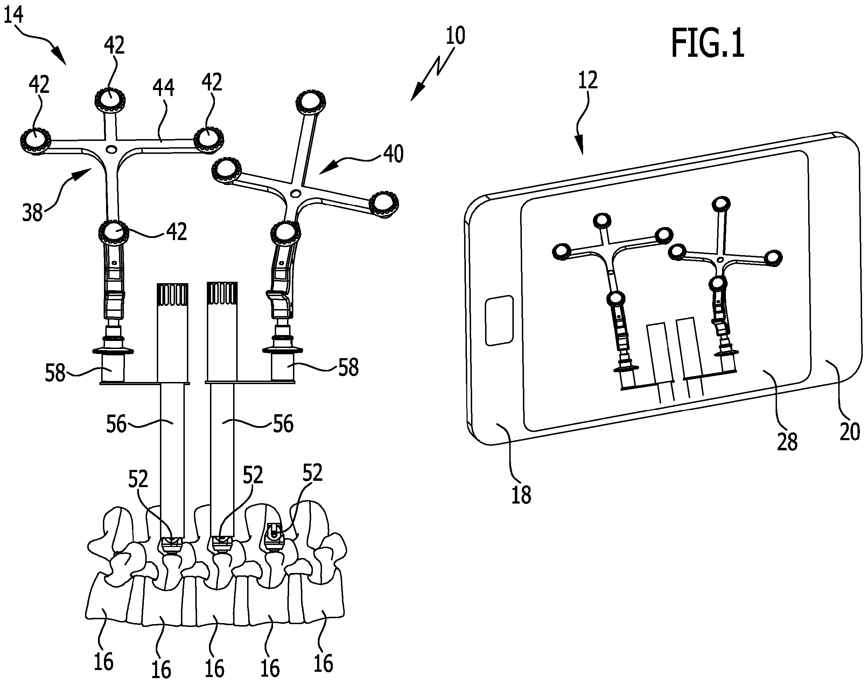

Figur 1 zeigt in schematischer perspektivischer Darstellung eine mit dem Bezugszeichen 10 belegte vorteilhafte Ausführungsform eines erfindungsgemäßen medizinischen Instrumentariums. Das Instrumentarium 10 umfasst ein medizintechnisches Navigationssystem 12 und ein chirurgisches Fixationssystem 14, das in Figur 1 nur teilweise dargestellt ist. Figur 1 zeigt ferner eine Mehrzahl von relativ zueinander zu stabilisierenden Wirbeln 16.

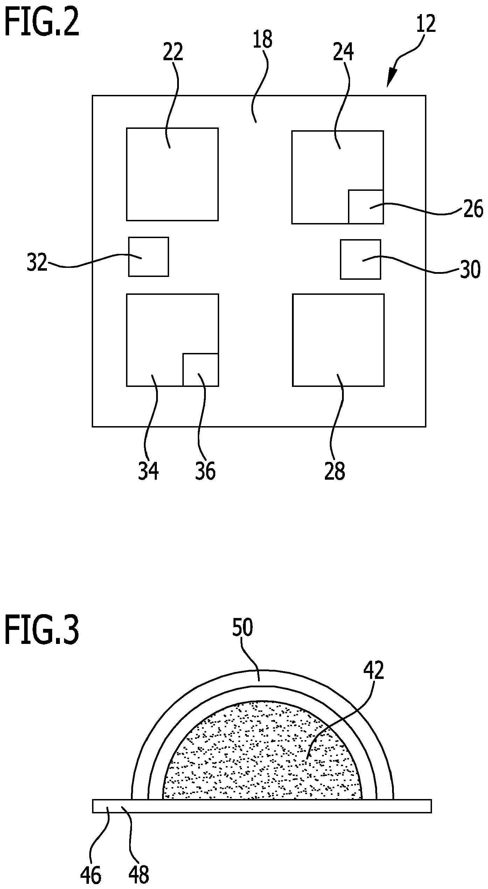

Das Navigationssystem 12, siehe auch Figur 2, ist ein handhaltbares integriertes Navigationssystem. "Integriert" ist vorliegend insbesondere derart aufzufassen, dass alle Komponenten des Navigationssystems 12 in einem gemeinsamen Gehäuse 18 angeordnet sind. Das Navigationssystem 12 ist insbesondere ausgestaltet als Tablet-Computer oder, im vorliegenden Fall, als Smartphone 20. Das Smartphone 20 kann von einem Operateur handhabungsfreundlich bedient und im Raum bevorzugt frei bewegt werden.

Das Navigationssystem 12 umfasst eine Datenverarbeitungseinheit 22, die zum Beispiel einen Mikroprozessor umfasst oder als solcher ausgestaltet ist. Durch die Datenverarbeitungseinheit 22 ist ein Datenverarbeitungsprogramm ausführbar, mit dem optische Aufnahmen einer Erfassungseinheit 24 des Navigationssystems 12 analysiert werden können. Dementsprechend umfasst das Datenverarbeitungsprogramm insbesondere Algorithmen der Bildverarbeitung. Die Erfassungseinheit 24 umfasst eine und bevorzugt genau eine digitale Kamera 26.

Außer mit der Erfassungseinheit 24 steht die Datenverarbeitungseinheit 22 auch mit einer Anzeigeeinheit 28, einer Sensoreinheit 30 und einer Kommunikationsschnittstelle 32 des Navigationssystems 12 in elektrischer Verbindung.

Die Anzeigeeinheit 28 ist insbesondere als Touchscreen ausgestaltet.

Die Sensoreinheit 30 umfasst einen Neigungssensor, mit dem eine Neigung des Navigationssystems 12 in mehreren Raumrichtungen in einem absoluten Bezugssystem ermittelbar ist, speziell einem Welt-Koordinatensystem. Dadurch ist es zum Beispiel möglich, die Neigung des Navigationssystems 12 relativ zur Horizontalebene zu ermitteln.

Über die Kommunikationsschnittstelle 32 können vom Navigationssystem 12 Daten an einen externen, räumlich getrennten Empfänger übertragen werden. Vorzugsweise handelt es sich um eine drahtlose Kommunikationsschnittstelle 32. Die Daten können insbesondere Bilder der Kamera 26 umfassen sowie ferner Lage- und/oder Orientierungsdaten von medizinischen Markiereinrichtungen, von diesen referenzierten charakteristischen Punkten, Bezugspunkten oder anatomischen Landmarken.

Das Navigationssystem 12 umfasst ferner eine Beleuchtungseinheit 34, um ein Sichtfeld der Kamera 26 zumindest teilweise auszuleuchten. Die Beleuchtungseinheit 34 weist vorzugsweise mindestens eine LED-Lichtquelle 36 auf. Günstigerweise emittiert die Beleuchtungseinheit 34 Licht im sichtbaren Spektralbereich von ungefähr 400 nm bis ungefähr 800 nm oder eines Teils davon.

Das Instrumentarium 10 umfasst mindestens eine medizinische Markiereinrichtung 38. Die Markiereinrichtung 38 kann Bestandteil des Fixationssystems 14 sein. Vorliegend sind zwei Markiereinrichtungen 38, 40 vorgesehen. In funktioneller Hinsicht sind die Markiereinrichtungen 38 und 40 identisch, jedoch in räumlicher Ausgestaltung unterschiedlich. Dies gibt die Möglichkeit, beide Markiereinrichtungen 38, 40 getrennt oder simultan mit dem Navigationssystem 12 zu erfassen, zu identifizieren und in Lage und/oder Orientierung im Raum zu verfolgen.

Die Markiereinrichtungen 38, 40 umfassen jeweils eine Mehrzahl von medizinischen Markierelementen 42, schematisch teilweise in Figur 3 dargestellt. Die Markierelemente 42 sind an einem Halteelement 44 festgelegt. Die Markierelemente 42 sind dazu ausgebildet, das von der LED-Lichtquelle 36 emittierte Licht zu reflektieren. Auf diese Weise können Signalanteile der Markierelemente 42 von der Kamera 26 zuverlässig erfasst und von der Datenverarbeitungseinheit 22 zuverlässig identifiziert werden.

Zur Erhöhung des Kontrastes zwischen den Markierelementen 42 und dem Halteelement 44 umfassen die Markiereinrichtungen 38, 40 jeweilige Kontrasterhöhungselemente 46. Die Kontrasterhöhungselemente 46 sind ausgestaltet als die Ringe 48. Die Reflektivität der Ringe 48 für das von der LED-Lichtquelle 36 emittierte Licht ist geringer als die Reflektivität der Markierelemente 42.

Die Ringe 48 umgeben die Markierelemente 42 sowie ein jeweiliges diesen zugeordnetes Schutzelement 50. Die Schutzelemente 50 sind kugelkalottenförmig und umgeben die Markierelemente 42. Die Schutzelemente 50 weisen dadurch eine haubenförmige Gestalt auf. Sie sind transparent für das von der LED-Lichtquelle 36 emittierte Licht und schützen die Markierelemente 42 vor Verschmutzung, zum Beispiel durch Blut.

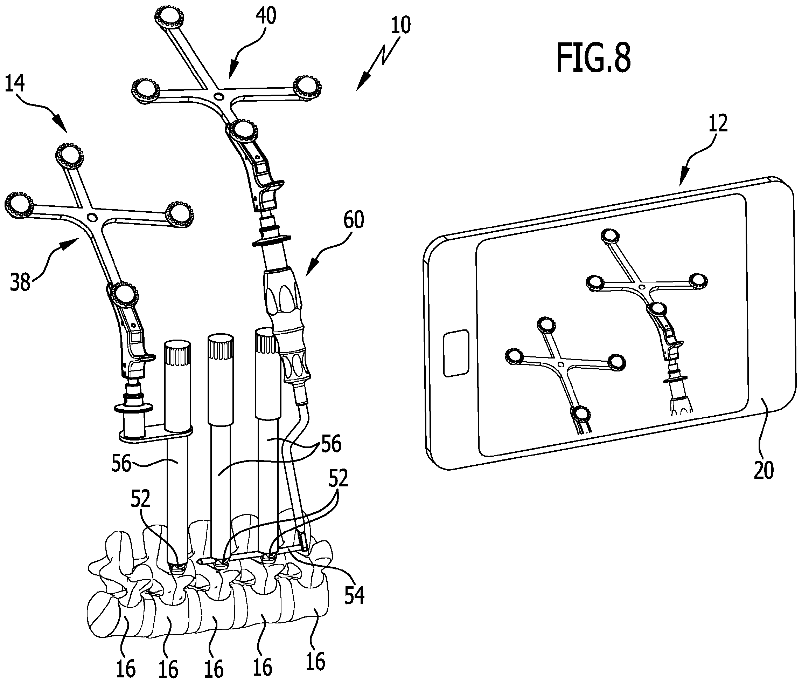

Das Fixationssystem 14 dient zur Stabilisierung der Wirbel 16 relativ zueinander. Zu diesem Zweck umfasst das Fixationssystem 14 in an sich bekannter Weise Verankerungselemente in Gestalt von Knochenschrauben 52. Die Knochenschrauben 52 können in den jeweiligen Wirbeln 16 fixiert werden. Ferner weist das Navigationssystem 12 ein Verbindungselement (Figur 8) in Gestalt eines Stabes 54 auf. Der Stab 54 kann klemmend an den Knochenschrauben fixiert werden.

Weiter umfasst das Fixationssystem 14 Verlängerungselemente in Gestalt von Verlängerungstuben 56. Die Verlängerungstuben 56 können in an sich bekannter Weise kraft- und/oder formschlüssig mit den Knochenschrauben 52 verbunden werden. Vorzugsweise kann perkutan auf die Knochenschrauben 52 eingewirkt werden.

Die Markiereinrichtungen 38, 40 können lösbar und selektiv an den Verlängerungstuben 56 festgelegt werden. Zu diesem Zweck ist beispielsweise ein an der Verlängerungstube 56 gehaltenes Adapterelement 58 vorgesehen.

Die Geometrie des Adapterelementes 58 und der Verlängerungstube 56 ist in dem Navigationssystem 12 gespeichert. Durch Erfassen der Lage- und/oder Orientierung einer der Markiereinrichtungen 38, 40 kann dadurch auf Lage und Orientierung der Knochenschraube 52 geschlossen werden, mit der die entsprechende Verlängerungstube 56 verbunden ist.

Nachfolgend wird die Funktionsweise und die Verwendung des Instrumentariums 10 erläutert, insbesondere des Navigationssystems 12.

Mit dem Navigationssystem 12 werden Bezugspunkte, die durch die Knochenschrauben 52 definiert werden, in einem Referenzkoordinatensystem ermittelt. Die Knochenschrauben 52 sind dementsprechend Referenzelemente. Das Referenzkoordinatensystem wird durch eine der Markiereinrichtungen 38, 40 definiert, vorliegend beispielsweise durch die Markiereinrichtung 38.

Der Operateur nimmt mit der Kamera 26 des Navigationssystems 12 ein Bild des Operationsgebietes auf, das insbesondere die Markiereinrichtungen 38, 40 umfasst (Figur 1). Die Markiereinrichtung 38 ist mit einer der Knochenschrauben 52 gekoppelt, die Markiereinrichtung 40 mit der daneben liegenden Knochenschraube.

Im Bild der Kamera 26 können die Markierelemente 42 aufgrund ihrer reflektierenden Eigenschaft und der Beleuchtung mittels der LED-Lichtquelle 36 zuverlässig erkannt und dadurch die Lage und die Orientierung der Markiereinrichtungen 38, 40 von der Datenverarbeitungseinheit 22 mittels Bildverarbeitungsalgorithmen festgestellt werden. Dies gibt die Möglichkeit, einen Raumvektor von den durch die Knochenschrauben 52 definierten Bezugspunkten im Referenzkoordinatensystem zu ermitteln.

Besonders vorteilhaft ist es dabei, dass unter Berücksichtigung eines Signals der Sensoreinheit 30 festgestellt werden kann, wie die Orientierung des Raumvektors im absoluten Koordinatensystem verläuft. Dies ist ohne a priori Kenntnis über die Lage und Orientierung des Patienten möglich und ohne dass dieser hierfür fixiert zu sein braucht.

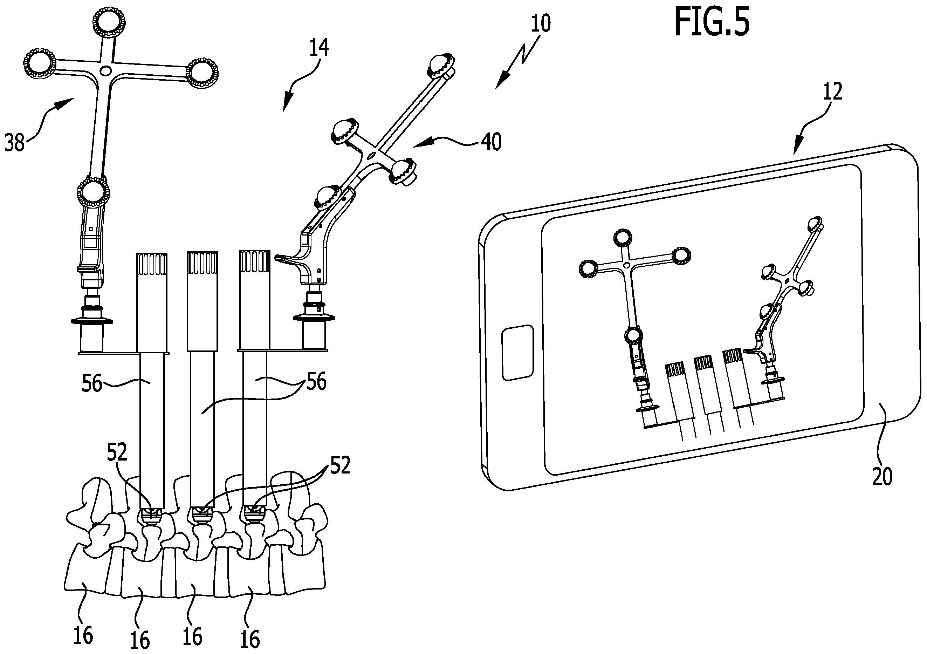

Im weiteren Verlauf des Eingriffs kann die Markiereinrichtung 40 mit einer weiteren Verlängerungstube 56 verbunden werden. Die weitere Verlängerungstube 56 ist mit der nächsten der Knochenschrauben 52 gekoppelt (Figur 5). Die Markiereinrichtung 38 bleibt mit der ersten Knochenschraube 52 gekoppelt. Das Referenzkoordinatensystem wird weiterhin durch die Markiereinrichtung 38 definiert, und auch die Lage der weiteren Knochenschraube 52, die nunmehr durch die Markiereinrichtung 40 referenziert wird, kann im Referenzkoordinatensystem ermittelt werden.

Mit der Kamera 26 wird ein weiteres Bild des Operationsgebietes aufgenommen, das die Markiereinrichtungen 38 und 40 umfasst. Denkbar ist auch, dass nicht Einzelbilder aufgenommen werden, sondern eine Bildfolge in Form eines Videos. Mit dem bzw. den weiteren Bild(ern) kann der weitere Raumvektor zwischen den Bezugspunkten wie durch die Knochenschrauben 52 definiert im Referenzkoordinatensystem ermittelt werden.

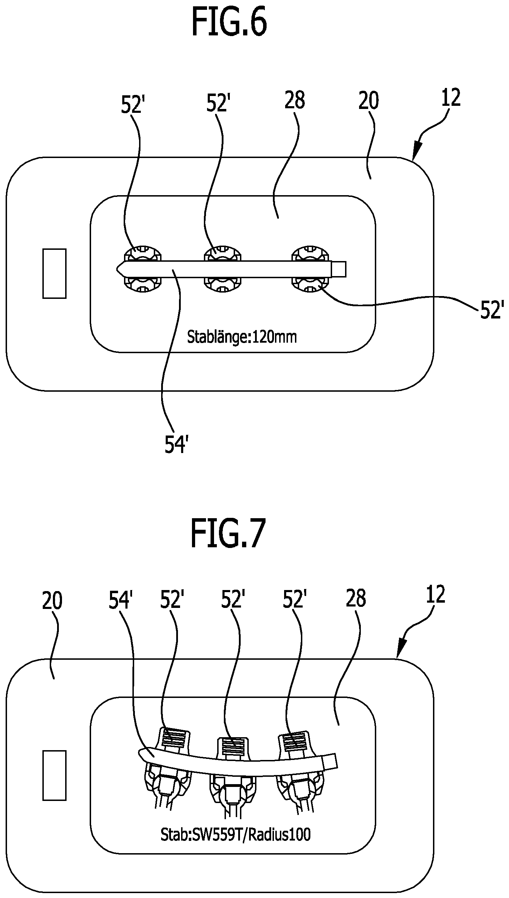

Aus den Raumvektoren kann ein (vorliegend offener) Polygonzug der Bezugspunkte im Referenzkoordinatensystem errechnet werden. Basierend auf diesen Informationen kann die Datenverarbeitungseinheit 42 dem Operateur an der Anzeigeeinheit 28 einen Vorschlag für einen zu verwendenden Stab 54 unterbreiten, mit dem die Knochenschrauben 52 in der gewünschten Relativorientierung fixiert werden können. Die Figuren 6 und 7 zeigen beispielhaft die Länge des vorgeschlagenen Stabes 54 bzw. dessen Typ und dessen Stabkrümmung.

Das Navigationssystem 12 kann auch bei der Implantation des Stabes 54 unterstützend eingesetzt werden (Figuren 8 bis 10). Zur Implantation des Stabes 54 wird dieser an einem Einsetz- oder Implantationswerkzeug 60 festgelegt. Eine Markiereinrichtung, vorliegend die Markiereinrichtung 40, wird mit dem Implantationswerkzeug 60 verbunden. Aufgrund der bekannten Geometrie des Implantationswerkzeuges 60 und des Stabes 54 kann die Lage und Orientierung des Stabes 54 im Referenzkoordinatensystem ermittelt werden. Das Referenzkoordinatensystem wird weiter durch die Markiereinrichtung 38 definiert, die an der ersten der Verlängerungstuben 56 und an der ersten Knochenschraube 52 fixiert bleibt.

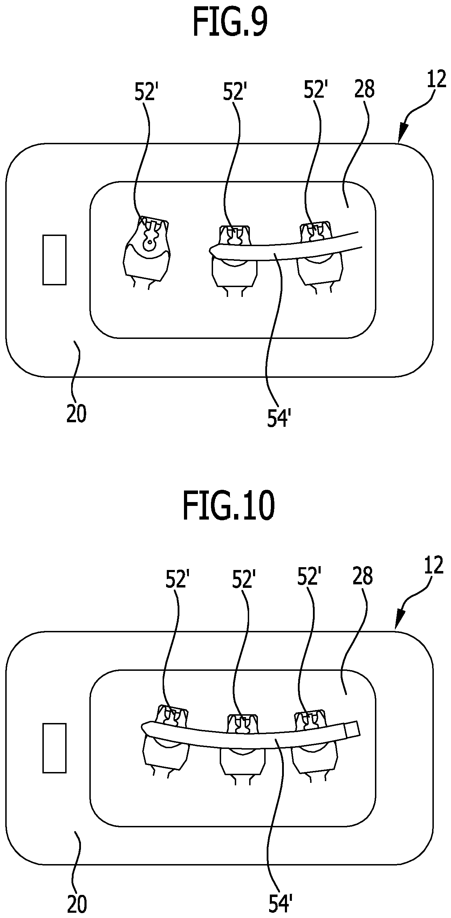

Der Benutzer erstellt mit der Kamera 26 weitere Bilder oder Bildfolgen vom Operationsgebiet, in denen die Markiereinrichtungen 38 und 40 erfasst werden. Mittels Bildverarbeitung kann die Datenverarbeitungseinheit 22 die Lage und Orientierung des Stabes 54 relativ zu den Knochenschrauben 52 ermitteln. Die Bedienperson kann über die Anzeigeeinheit 28 zum Führen des Implantationswerkzeuges 60 angeleitet werden. Die Figuren 9 und 10 stellen dies schematisch dar. Symbole des Stabes 54 und der Knochenschrauben 52 können an der Anzeigeeinheit 28 eingeblendet werden. Anhand dieser Symbole kann der Benutzer das Implantationswerkzeug 60 so führen, dass der Stab 54 durch an den Knochenschrauben 52 angeordnete Einführöffnungen geführt wird. Die Hinweise können anstelle des von der Kamera 26 erstellten Bildes eingeblendet werden oder dieses überblenden.

Die Symbole der Knochenschrauben 52 und des Stabes 54 an der Anzeigeeinheit 28 sind in der Zeichnung mit demselben Bezugszeichen und zusätzlichem Hochkomma (') gekennzeichnet.

Über die Kommunikationsschnittstelle 32 können, wie erwähnt, während des Eingriffs erfasste Informationen wie Bezugspunkte, anatomische Landmarken oder insbesondere auch Bilder der Kamera 26 an einen externen Empfänger übertragen werden. Dies dient zum Beispiel zur Dokumentation des Eingriffs oder für Schulungszwecke.

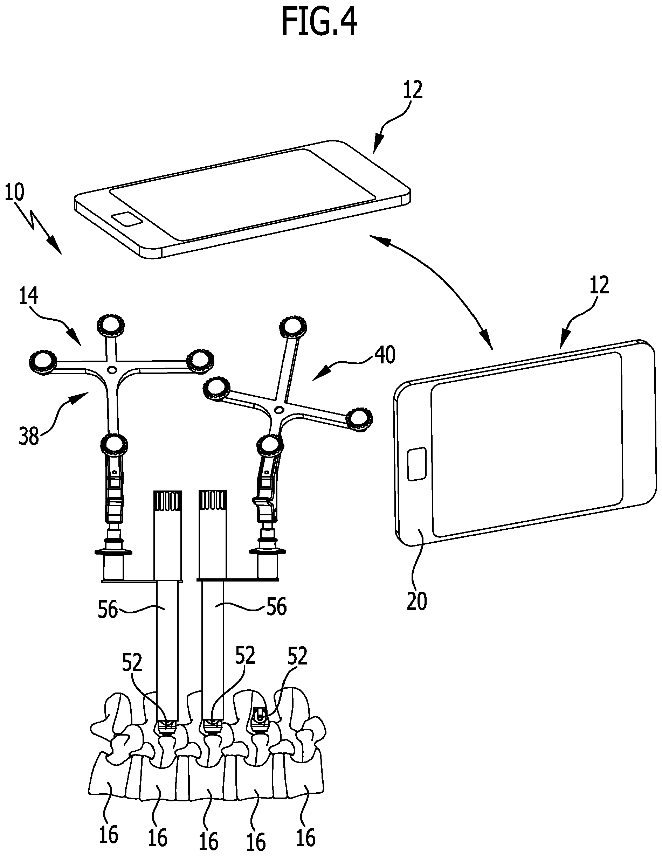

Beim Instrumentarium 10 erweist es sich als besonders günstig, dass das Navigationssystem 12 integriert und handhaltbar und frei von einer räumlichen Fixierung ist. Dies gibt dem Operateur die Möglichkeit, das Navigationssystem 12 frei im Raum zu bewegen, um möglichst gute Aufnahmen des Operationsgebietes zu erstellen. Dies ist beispielhaft in Figur 4 dargestellt, die das Navigationssystem 12 in zwei unterschiedlichen Relativorientierungen zum Fixationssystem 14 zeigt.

### Bezugszeichenliste:

- 10: Instrumentarium
- 12: Navigationssystem
- 14: Fixationssystem
- 16: Wirbel
- 18: Gehäuse
- 20: Smartphone
- 22: Datenverarbeitungseinheit
- 24: Erfassungseinheit
- 26: Kamera
- 28: Anzeigeeinheit
- 30: Sensoreinheit
- 32: Kommunikationsschnittstelle
- 34: Beleuchtungseinheit
- 36: LED-Lichtquelle
- 38: Markiereinrichtung
- 40: Markiereinrichtung
- 42: Markierelement
- 44: Halteelement
- 46: Kontrasterhöhungselement
- 48: Ring
- 50: Schutzelement
- 52: Knochenschraube
- 54: Stab
- 56: Verlängerungstube
- 58: Adapterelement
- 60: Implantationswerkzeug

## Patentansprüche

1. Medizinisches Instrumentarium, umfassend ein handhaltbares integriertes medizintechnisches Navigationssystem (12), das eine eine Kamera (26) aufweisende optische Erfassungseinheit (24) aufweist, eine Datenverarbeitungseinheit (22) und eine optische Anzeigeeinheit (28), wobei die Datenverarbeitungseinheit (22) mit der Erfassungseinheit (24) und der Anzeigeeinheit (28) gekoppelt ist, wobei Lage- und/oder Orientierungsdaten einer medizinischen Markiereinrichtung (38, 40), die mit der Erfassungseinheit (24) erfassbar ist, von der Datenverarbeitungseinheit (22) verarbeitbar sind und diesbezügliche Informationen an der Anzeigeeinheit (28) darstellbar sind, wobei das Instrumentarium (10) eine Beleuchtungseinheit (34) aufweist, mit der Licht in Richtung der mit der Erfassungseinheit (24) erfassbaren Markiereinrichtung (38, 40) aussendbar ist, wobei das Instrumentarium (10) zwei oder mehr medizinische Markiereinrichtungen (38, 40) aufweist, die jeweils eine Mehrzahl von Markierelementen (42) aufweisen, die das Licht der Beleuchtungseinheit (34) retroreflektierend ausgestaltet sind, wobei mit der Datenverarbeitungseinheit (22) ein Raumvektor zwischen von den Markiereinrichtungen (38, 40) referenzierten Bezugspunkten ermittelbar ist, wobei eine der Markiereinrichtungen (38, 40) als Referenzmarkiereinrichtung (38, 40) zur Definition eines Referenzkoordinatensystems heranziehbar ist, wobei das Navigationssystem (12) eine Sensoreinheit (30) aufweist zum Bereitstellen eines Signals über die Neigung des Navigationssystems (12) in mindestens einer Raumrichtung eines absoluten Bezugssystems, und wobei mit der Datenverarbeitungseinheit (22) die Orientierung des Raumvektors im absoluten Bezugssystem ermittelbar ist, und wobei anhand der Orientierungen einer Mehrzahl von zwei oder mehr Raumvektoren eine Neigung einer durch diese definierten Ebene relativ zu einer Bezugsebene, insbesondere einer Horizontalebene, im absoluten Bezugssystem ermittelbar ist.

2. Instrumentarium nach Anspruch 1, **dadurch gekennzeichnet, dass** das Navigationssystem (12) als Smartphone (20) oder als Tablet-Computer ausgestaltet ist.

3. Instrumentarium nach Anspruch 1 oder 2, **dadurch gekennzeichnet, dass** das Navigationssystem (12) die Beleuchtungseinheit (34) umfasst.

4. Instrumentarium nach einem der voranstehenden Ansprüche, **dadurch gekennzeichnet, dass** die Beleuchtungseinheit (34) mindestens eine LED-Lichtquelle (36) aufweist.

5. Instrumentarium nach einem der voranstehenden Ansprüche, **dadurch gekennzeichnet, dass** sichtbares Licht von der Beleuchtungseinheit (34) emittierbar ist.

6. Instrumentarium nach einem der voranstehenden Ansprüche, **dadurch gekennzeichnet, dass** die Erfassungseinheit (24) genau eine Kamera (26) aufweist.

7. Instrumentarium nach einem der voranstehenden Ansprüche, **dadurch gekennzeichnet, dass** an der Anzeigeeinheit (28) ein mit der Kamera (26) erfassbares Bild darstellbar ist.

8. Instrumentarium nach einem der voranstehenden Ansprüche, **dadurch gekennzeichnet, dass** das Navigationssystem (12) eine Kommunikationsschnittstelle (32) aufweist zum Übertragen von Daten an eine externe Empfangseinheit, insbesondere Bildern, die mit der Kamera (26) aufgenommen werden oder wurden.

9. Instrumentarium nach einem der voranstehenden Ansprüche, **dadurch gekennzeichnet, dass** das Navigationssystem (12) im Raum frei beweglich und frei von einer mechanischen Kopplung mit einem chirurgischen Instrument oder einem Implantat ist.

10. Instrumentarium nach einem der voranstehenden Ansprüche, **dadurch gekennzeichnet, dass** die Markiereinrichtungen (38, 40) simultan mit der Erfassungseinheit (24) erfassbar sind und der Raumvektor mit der Datenverarbeitungseinheit (22) anhand eines Bildes der Kamera (26) ermittelbar ist.

11. Instrumentarium nach einem der voranstehenden Ansprüche, **dadurch gekennzeichnet, dass** das Instrumentarium (10) zwei oder mehr zu referenzierende Referenzelemente umfasst und dass die Markiereinrichtungen (38, 40) selektiv mit den Referenzelementen koppelbar sind und anhand der Datenverarbeitungseinheit (22) die Relativposition der Referenzelemente im Referenzkoordinatensystem ermittelbar ist.

12. Instrumentarium nach Anspruch 11, **dadurch gekennzeichnet, dass** die Referenzelemente Knochenschrauben (52) sind oder solche umfassen, und dass die Markiereinrichtungen (38, 40) mit Verlängerungselementen, insbesondere Verlängerungstuben (56), vorzugsweise perkutan mit den Knochenschrauben (52) koppelbar sind.

13. Instrumentarium nach Anspruch 11 oder 12, **dadurch gekennzeichnet, dass** das Instrumentarium (10) ein Verbindungselement aufweist zum Verbinden der Referenzelemente, und dass von der Datenverarbeitungseinheit (22) die Geometrie des Verbindungselementes ermittelbar ist, um die Referenzelemente zu verbinden, vorzugsweise dass an der Anzeigeeinheit (28) ein Hinweis betreffend das Verbindungselement bereitstellbar ist.

14. Instrumentarium nach Anspruch 13, **dadurch gekennzeichnet, dass** das Verbindungselement mit einer der Markiereinrichtungen (38, 40) koppelbar ist und von der Datenverarbeitungseinheit (22) die Lage des Verbindungselementes relativ zu den Referenzelementen ermittelbar und einem Benutzer Hinweise an der Anzeigeeinheit (28) zum Führen des Verbindungselementes bereitstellbar sind.

15. Instrumentarium nach Anspruch 13 oder 14, **dadurch gekennzeichnet, dass** das Verbindungselement ein Stab ist (54) und dass eine der Markiereinrichtungen (40) an einem Einsetzwerkzeug (60) zum Führen des Stabes (54) festgelegt oder festlegbar ist.

## Claims

1. Medical instrumentation, comprising a hand-held, integrated, medical navigation system (12) which comprises an optical detection unit (24) having a camera (26), a data processing unit (22) and an optical display unit (28), the data processing unit (22) being coupled to the detection unit (24) and the display unit (28), it being possible for location and/or orientation data of a medical marking device (38, 40) which is detectable with the detection unit (24) to be processed by the data processing unit (22) and information relating thereto to be represented on the display unit (28), the instrumentation (10) comprising an illumination unit (34) with which light is emittable in the direction of the marking device (38, 40) detectable with the detection unit (24), wherein the instrumentation (10) comprises two or more medical marking devices (38, 40) which each have a plurality of marking elements (42) which are configured for retroflection of the light of the illumination unit (34), wherein a space vector between reference points referenced by the marking devices (38, 40) is determinable with the data processing unit (22), with one of the marking devices (38, 40) being usable as reference marking device (38, 40) for defining a reference coordinate system, wherein the navigation system (12) comprises a sensor unit (30) for providing a signal relating to the inclination of the navigation system (12) in at least one direction in space of an absolute reference system, and wherein the orientation of the space vector is determinable with the data processing unit (22) in the absolute reference system, and wherein on the basis of the orientations of a plurality of two or more space vectors, an inclination of a plane defined by these relative to a reference plane, in particular, a horizontal plane, is determinable in the absolute reference system.

2. Instrumentation in accordance with claim 1, **characterized in that** the navigation system (12) is configured as smartphone (20) or as tablet computer.

3. Instrumentation in accordance with claim 1 or 2, **characterized in that** the navigation system (12) comprises the illumination unit (34).

4. Instrumentation in accordance with any one of the preceding claims, **characterized in that** the illumination unit (34) comprises at least one LED light source (36).

5. Instrumentation in accordance with any one of the preceding claims, **characterized in that** visible light is emittable by the illumination unit (34).

6. Instrumentation in accordance with any one of the preceding claims, **characterized in that** the detection unit (24) comprises precisely one camera (26).

7. Instrumentation in accordance with any one of the preceding claims, **characterized in that** an image recordable with the camera (26) is representable on the display unit (28).

8. Instrumentation in accordance with any one of the preceding claims, **characterized in that** the navigation system (12) comprises a communication interface (32) for transmitting data to an external receiving unit, in particular, images which are or were taken with the camera (26).

9. Instrumentation in accordance with any one of the preceding claims, **characterized in that** the navigation system (12) is freely movable in space and is free of any mechanical coupling with a surgical instrument or an implant.

10. Instrumentation in accordance with any one of the preceding claims, **characterized in that** the marking devices (38, 40) are simultaneously detectable with the detection unit (24), and the space vector is determinable with the data processing unit (22) on the basis of an image of the camera (26).

11. Instrumentation in accordance with any one of the preceding claims, **characterized in that** the instrumentation (10) comprises two or more reference elements to be referenced, and **in that** the marking devices (38, 40) are adapted for selective coupling to the reference elements, and the relative position of the reference elements is determinable by means of the data processing unit (22) in the reference coordinate system.

12. Instrumentation in accordance with claim 11, **characterized in that** the reference elements are or comprise bone screws (52), and **in that** the marking devices (38, 40) with extension elements, in particular, extension tubes (56), are adapted for coupling, preferably percutaneously, to the bone screws (52).

13. Instrumentation in accordance with claim 11 or 12, **characterized in that** the instrumentation (10) comprises a connecting element for connecting the reference elements, and **in that** the geometry of the connecting element is determinable by the data processing unit (22) in order to connect the reference elements, preferably **in that** an indication relating to the connecting element is providable on the display unit (28).

14. Instrumentation in accordance with claim 13, **characterized in that** the connecting element is adapted for coupling to one of the marking devices (38, 40), and the position of the connecting element relative to the reference elements is determinable by the data processing unit (22) and indications are providable for a user on the display unit (28) for guiding the connecting element.

15. Instrumentation in accordance with claim 13 or 14, **characterized in that** the connecting element is (54) a rod, and **in that** the marking device (40) is fixed or fixable to an insertion tool (60) for guiding the rod (54).

## Revendications

1. Instrumentation médicale, comprenant un système de navigation (12) de technologie médicale, intégré et pouvant être tenu à la main, qui comporte une unité optique d'acquisition de données (24) présentant une caméra (26), une unité de traitement de données (22) et une unité de visualisation optique (28), instrumentation dans laquelle l'unité de traitement de données (22) est couplée à l'unité d'acquisition de données (24) et à l'unité de visualisation (28),
dans laquelle des données de position et/ou d'orientation d'un dispositif de marquage médical (38, 40), qui peut être détecté avec l'unité d'acquisition de données (24), peuvent être traitées par l'unité de traitement de données (22), et des informations à cet égard peuvent être représentées sur l'unité de visualisation (28),
dans laquelle l'instrumentation (10) comporte une unité d'éclairage (34) à l'aide de laquelle peut être émise de la lumière en direction du dispositif de marquage (38, 40) pouvant être détecté avec l'unité d'acquisition (24),
dans laquelle l'instrumentation (10) comprend deux dispositifs de marquage médical (38, 40) ou davantage, qui présentent chacun respectivement une pluralité d'éléments de marquage (42) de configuration rétroréfléchissante pour la lumière de l'unité d'éclairage (34),
dans laquelle à l'aide de l'unité de traitement de données (22), il est possible de déterminer un vecteur spatial entre des points de référence référencés par les dispositifs de marquage (38, 40),
dans laquelle l'un des dispositifs de marquage (38, 40) peut être mis à contribution en tant que dispositif de marquage de référence (38, 40) pour définir un système de coordonnées de référence,
dans laquelle le système de navigation (12) comprend une unité de capteur (30) pour fournir un signal relatif à l'inclinaison du système de navigation (12) dans au moins une direction spatiale d'un système de référence absolu, et
dans laquelle à l'aide de l'unité de traitement de données (22), il est possible de déterminer l'orientation du vecteur spatial dans le système de référence absolu, et
dans laquelle au regard de l'orientation d'une pluralité de deux vecteurs spatiaux ou davantage, il est possible de déterminer une inclinaison d'un plan défini par ceux-ci, par rapport à un plan de référence, notamment un plan horizontal, dans le système de référence absolu.

2. Instrumentation selon la revendication 1, **caractérisée en ce que** le système de navigation (12) est réalisé sous forme de smartphone (20) ou d'ordinateur-tablette.

3. Instrumentation selon la revendication 1 ou la revendication 2, **caractérisée en ce que** le système de navigation (12) comprend l'unité d'éclairage (34).

4. Instrumentation selon l'une des revendications précédentes, **caractérisée en ce que** l'unité d'éclairage (34) comporte au moins une source de lumière à LED (36).

5. Instrumentation selon l'une des revendications précédentes, **caractérisée en ce que** l'unité d'éclairage (34) est en mesure d'émettre de la lumière visible.

6. Instrumentation selon l'une des revendications précédentes, **caractérisée en ce que** l'unité d'acquisition de données (24) comporte exactement une caméra (26).

7. Instrumentation selon l'une des revendications précédentes, **caractérisée en ce que** sur l'unité de visualisation (28) peut être représentée une image pouvant être relevée à l'aide de la caméra (26).

8. Instrumentation selon l'une des revendications précédentes, **caractérisée en ce que** le système de navigation (12) comporte une interface de communication (32) pour transmettre à une unité de réception externe, des données, notamment des images, qui sont relevées ou ont été relevées à l'aide de la caméra (26).

9. Instrumentation selon l'une des revendications précédentes, **caractérisée en ce que** le système de navigation (12) est librement mobile dans l'espace et est libre de tout couplage mécanique avec un instrument chirurgical ou un implant.

10. Instrumentation selon l'une des revendications précédentes, **caractérisée en ce que** les dispositifs de marquage (38, 40) peuvent être détectés simultanément avec l'unité d'acquisition de données (24), et le vecteur spatial peut être déterminé à l'aide de l'unité de traitement de données (22) au regard d'une image de la caméra (26).

11. Instrumentation selon l'une des revendications précédentes, **caractérisée en ce que** l'instrumentation (10) comprend deux éléments de référence à référencer ou davantage, et **en ce que** les dispositifs de marquage (38, 40) peuvent être couplés sélectivement avec les éléments de référence, et à l'aide de l'unité de traitement de données (22), il est possible de déterminer la position relative des éléments de référence dans le système de coordonnées de référence.

12. Instrumentation selon la revendication 11, **caractérisée en ce que** les éléments de référence sont des vis à os (52) ou comportent de telles vis, et **en ce que** les dispositifs de marquage (38, 40) peuvent être couplés aux vis à os (52), de préférence par voie percutanée, à l'aide d'éléments prolongateurs, notamment des tubes prolongateurs (56).

13. Instrumentation selon la revendication 11 ou la revendication 12, **caractérisée en ce que** l'instrumentation (10) comporte un élément de liaison pour relier les éléments de référence, et **en ce que** l'unité de traitement de données (22) permet de déterminer la géométrie de l'élément de liaison en vue de relier les éléments de référence, de préférence **en ce qu'**il est possible de fournir sur l'unité de visualisation (28), une indication concernant l'élément de liaison.

14. Instrumentation selon la revendication 13, **caractérisée en ce que** l'élément de liaison peut être couplé à l'un des dispositifs de marquage (38, 40), et l'unité de traitement de données (22) est en mesure de déterminer la position de l'élément de liaison par rapport aux éléments de référence, et de fournir à un utilisateur, sur l'unité de visualisation (28), des indications pour guider l'élément de liaison.

15. Instrumentation selon la revendication 13 ou la revendication 14, **caractérisée en ce que** l'élément de liaison est une tige (54), et **en ce que** l'un des dispositifs de marquage (40) est fixé ou peut être fixé à un outil d'insertion (60) pour guider la tige (54).
